# EUROPÄISCHE PATENTSCHRIFT

(11) **EP 0 550 895 B1**
(45) Veröffentlichungstag und Bekanntmachung des Hinweises auf die Patenterteilung: **16.07.1997**
(21) Anmeldenummer: 92122011.7
(22) Anmeldetag: 24.12.1992
(51) Int. Cl.: C07H 17/08, A61K 31/70

(54) **4,13-Dioxabicyclo[8.2.1]tridecenon-Derivate, Verfahren und Zwischenprodukte zu ihrer Herstellung und diese Verbindungen enthaltende Arzneimittel**
4,13-dioxabicyclo[8.2.1]tridecenon-derivatives, method and intermediates for their preparation, and medicaments containing the same
Dérivés de 4,13-dioxabicyclo[8.2.1]tridecenon, leur procédé et les intermédiaires de préparation et médicaments les renfermant

(30) Priorität: 07.01.1992 DE 4200145
(43) Veröffentlichungstag der Anmeldung: 14.07.1993
(73) Patentinhaber: Solvay Pharmaceuticals GmbH, 30173 Hannover (DE)
(72) Erfinder: Höltje, Dagmar, W-3212 Gronau (DE); Preuschoff, Ulf, W-3110 Uelzen 5 (DE); Eeckhout, Christian, W-3280 Bad Pyrmont (DE)
(74) Vertreter: Lauer, Dieter, Dr.

(56) Entgegenhaltungen:
- EP-A- 0 296 717
- EP-A- 0 349 100
- EP-A- 0 382 472
- CHEMICAL AND PHARMACEUTICAL BULLETIN Bd. 37, Nr. 10, Oktober 1989, Tokyo, JP, T. TSUZUKI et al., Seiten 2687-2700
- THE JOURNAL OF ANTIBIOTICS, Bd. XL, Nr. 1, Januar 1987, Tokyo, JP, I.O. KIBWAGE et al., Seite 2

## Beschreibung

Die vorliegende Erfindung betrifft neue N-substituierte [2R,3R(2R',3R'),6R,7S,8S,9R,10R]-3-(2',3'-Dihydroxypent-2'-yl)-7-[(2,6-dideoxy-3-C-methyl-3-0-methyl-α-L-ribo-hexopyranosyl)-oxy]-9-[(3,4,6-trideoxy-3-amino-β-D-xylo-hexopyranosyl)-oxy]-2,6,8,10,12-pentamethyl-4,13-dioxabicyclo[8.2.1]tridec-12-en-5-on-Verbindungen mit motilin-agonistischen Eigenschaften und deren Säureadditionssalze sowie diese Verbindungen enthaltende pharmazeutische Zubereitungen und Verfahren und Zwischenprodukte zur Herstellung dieser Verbindungen. Die erfindungsgemäßen Verbindungen sind ringverengte N-Desmethyl-N-isopropyl-Derivate des Erythromycin A.

Das Antibiotikum Erythromycin A besitzt bekanntermaßen neben seinen antibiotischen Wirkungen auch für Antibiotika unerwünschte gastrointestinale Nebenwirkungen, u.a. eine starke Vermehrung der Kontraktionsaktivität im Magen-Darm-Bereich mit Magen- und Darmkrämpfen, Übelkeit, Erbrechen und Diarrhöe.

Es hat mehrere Versuche gegeben, das Erythromycin A so abzuwandeln, daß Derivate erhalten werden, in denen die antibiotische Wirkung praktisch nicht mehr vorhanden ist, jedoch eine die Motilität des gastrointestinalen Traktes beeinflussende Wirkung erhalten ist. Aus der EP-Patentanmeldung 0 349 100 A2 sind pharmazeutische Zusammensetzungen bekannt, welche als gastroprokinetischen Wirkstoff ein ringverengtes Erythromycin-A-Derivat oder dessen quartäre Salze enthalten, und welche die Magenmotilität durch cholinerge Mechanismen verstärken.

Der vorliegenden Erfindung liegt die Aufgabe zugrunde, neue ringverengte Derivate des Erythromycin A ohne Antibiotikawirkung und mit günstiger Wirkung auf die Motilität des gastrointestinalen Traktes zu entwickeln.

Es wurde nun gefunden, daß die neuen ringverengten N-Desmethyl-N-isopropyl-Derivate des Erythromycin A selektive motilin-agonistische Eigenschaften aufweisen und die Motilität des gastrointestinalen Traktes in günstiger Weise stimulieren und den Tonus des unteren Oesophagus Sphincter verstärkende Wirkungen zeigen. Aufgrund ihres Wirkungsprofils eignen sich die erfindungsgemäßen Substanzen zur Behandlung von Motilitätsstörungen im gastrointestinalen Trakt und zeichnen sich dabei durch eine gute Verträglichkeit aus.

Die vorliegende Erfindung betrifft daher neue [2R,3R(2-R',3R'),6R,7S,8S,9R,10R]-3-(2',3'-Dihydroxypent-2'-yl)2,6,8, 10,12-pentamethyl-4,13-dioxabicyclo[8.2.1]tridec-12-en-5-on-Derivate der allgemeinen Formel I
(s. Formel I)
worin R¹ Methyl bedeutet, und deren stabile und physiologisch verträgliche Säureadditionssalze.

Die Verbindungen der Formel I können erhalten werden, indem man auf an sich bekannte Weise in [2R,3R(2R',3R'),6R, 7S,8S,9R,10R]-3-(2',3'-Dihydroxypent-2'-yl)-2,6,8,10, 12-pentamethyl-4,13-dioxabicyclo[8.2.1]tridec-12-en-5-on-Derivate der allgemeinen Formel II
(s. Formel II)
worin R¹ Wasserstoff oder Methyl bedeutet, einen Isopropylrest einführt und in eine erhaltene Verbindung der Formel I, worin R¹ Wasserstoff bedeutet, einen Methylrest R¹ einführt und gewünschtenfalls freie Verbindungen der Formel I in ihre stabilen Säureadditionssalze überführt oder die Säureadditionssalze in die freien Verbindungen der Formel I überführt.

Zur Einführung des Isopropylrestes können die Verbindungen der Formel II auf an sich bekannte Weise alkyliert werden. Vorzugsweise wird die Alkylierung als reduktive Alkylierung auf an sich bekannte Weise durch Umsetzen der Verbindungen der Formel II mit Aceton unter reduzierenden Bedingungen durchgeführt. Beispielsweise können die Verbindungen der Formel II mit Aceton in Gegenwart eines Reduktionsmittels, beispielsweise einer komplexen Borhydridverbindung wie Natriumcyanoborhydrid, Natriumtriacetoxyborhydrid oder Natriumborhydrid, umgesetzt werden. Gewünschtenfalls kann die Alkylierung, insbesondere derjenigen Verbindung der Formel II, worin R¹ Methyl bedeutet, auch durch Umsetzen mit einem Isopropylhalogenid, insbesondere Isopropyljodid, oder Isopropylsulfat oder einem Isopropylsulfonsäure-ester erfolgen. Zweckmäßigerweise wird die Alkylierung in einem unter den Reaktionsbedingungen inerten organischen Lösungsmittel durchgeführt. Für die reduktive Alkylierung kann beispielsweise ein Überschuß an Aceton als Lösungsmittel dienen. Ferner eignen sich als Lösungsmittel auch cyclische Ether, wie Tetrahydrofuran oder Dioxan, aromatische Kohlenwasserstoffe wie Toluol oder auch niedere Alkohole. Die Alkylierung kann bei Temperaturen zwischen Raumtemperatur und Siedetemperatur des Lösungsmittels erfolgen. Bei der Alkylierung mit einem Isopropylderivat, beispielsweise einem Isopropylhalogenid wie Isopropyljodid, wird zweckmäßigerweise in Gegenwart einer Base wie beispielsweise einem Alkalimetallcarbonat oder einem tert. organischen Amin gearbeitet.

Die erhaltene Verbindung der Formel I, worin R¹ Wasserstoff bedeutet, kann nachträglich in an sich bekannter Weise zu der entsprechenden N-Methyl-Verbindung alkyliert werden. Die Alkylierung kann auf an sich bekannte Weise durch Umset zen mit einem Methylhalogenid oder als reduktive Alkylierung durch Umsetzen mit Formaldehyd unter reduzierenden Bedingungen erfolgen und kann beispielsweise bei den zur Alkylierung der Verbindungen der Formel II angegebenen Bedingungen durchgeführt werden.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden. Zur Vermeidung von Hydrolysenebenreaktionen ist es zweckmäßig, zur Salzbildung nur äquivalente Mengen Säuren zu verwenden.

Als pharmakologisch annehmbare Säureadditionssalze der Verbindungen der Formel I eignen sich beispielsweise deren Salze mit anorganischen Säuren, z.B. Kohlensäure, Halogenwasserstoffsäuren, insbesondere Chlorwasserstoffsäure, oder mit organischen Säuren, beispielsweise niederen aliphatischen Mono-oder Dicarbonsäuren wie Maleinsäure, Fumarsäure, Milchsäure, Weinsäure oder Essigsäure.

Die Verbindungen der Formel II können ausgehend von Erythromycin A der Formel III
(siehe Formel III)
nach an sich bekannten Methoden erhalten werden. So kann das Erythromycin A zunächst auf an sich bekannte Weise, beispielsweise nach dem aus der DE-OS 21 54 032 bekannten Verfahren, mono- oder didemethyliert werden durch Umsetzung mit Halogen, bevorzugt Jod, in einem inerten Lösungsmittel in Gegenwart einer geeigneten Base. Als Basen eignen sich beispielsweise Alkalimetallhydroxide, Alkalimetallcarbonate und Alkalimetallsalze von schwachen Carbonsäuren wie beispiels weise Alkalimetallacetate oder -propionate. Vorzugsweise werden 1 bis 5 Äquivalente des Halogens bezogen auf die Menge an zu demethylierender Erythromycin-Verbindung eingesetzt. Die Menge der Base wird vorzugsweise so gewählt, daß ein pH-Wert im Bereich von 5 bis 9 gewährleistet ist, um Hydrolyse- oder Alkoholyse-Nebenreaktionen zu vermeiden. Als Lösungsmittel eignen sich Methanol, cyclische Ether wie Dioxan oder Tetrahydrofuran, Dimethylformamid oder Mischungen der genannten Lösungsmittel mit Wasser. Die Demethylierung wird zweckmäßigerweise bei Temperaturen zwischen Raumtemperatur und 50 °C durchgeführt. Die Reaktion kann durch Bestrahlung mit Licht, z.B. Licht mit einer Wellenlänge von über 290 nm aus einer Quecksilberniederdrucklampe mit einem Filter aus Quarz oder hitzebeständigem Glas (z.B. Pyrex^{R}) gefördert werden. Die Reaktion erzeugt das monodemethylierte oder didemethylierte Produkt vorwiegend in Abhängigkeit von der Menge an verwendetem Halogen. Bei Verwendung von einem Äquivalent Halogen wird bevorzugt das monodemethylierte Produkt erhalten und bei Verwendung von 2 oder mehr Äquivalenten Halogen wird bevorzugt das didemethylierte Produkt erhalten. Gewünschtenfalls kann zur Herstellung des didemethylierten Produktes auch von bereits monodemethyiiertem Produkt ausgegangen werden.

Das mono- oder didemethylierte Erythromycin A kann auf an sich bekannte Weise durch milde Säurebehandlung in ein entsprechendes mono- oder didemethyliertes 8,9-Anhydroerythromycin-A-6,9-hemiketal der allgemeinen Formel IV
(s. Formel IV)
worin R¹ Wasserstoff oder Methyl bedeutet, überführt werden. Die Hemiketalbildung kann beispielsweise durch Behandeln mit Eisessig oder verdünnter Mineralsäure bei Temperaturen zwischen Raumtemperatur und ca. 50 °C erfolgen.

In den Verbindungen der Formel IV kann auf an sich bekannte Weise durch intramolekulare Translactonisierung eine Ringverengung des 14-gliedrigen Lactonringes des Erythromycin-Gerüstes zu einem 12-gliedrigen Lactonring unter Bildung der entsprechenden Verbindungen der Formel II durchgeführt werden. Hierzu werden die Verbindungen der Formel IV auf an sich bekannte Weise in einem niederen Alkohol in Gegenwart einer Base erhitzt, beispielsweise auf Temperaturen zwischen 40 °C und 70 °C, vorzugsweise Siedetemperatur des Reaktionsgemisches. Als Basen eignen sich insbesondere Alkalimetallcarbonate, aber auch organische Basen wie tertiäre Amine, insbesondere tertiäre Niederalkylamine. Bei dieser Ringverengung ändert sich die Konfiguration der Chiralitätszentren nicht.

Die neuen Verbindungen der Formel I und ihre physiologisch verträglichen Säureadditionssalze besitzen interessante pharmakologische Eigenschaften, insbesondere die Motilität des gastrointestinalen Traktes stimulierende motilin-agonistische Eigenschaften. Sie sind frei von antibiotischen Wirkungen und besitzen eine hohe selektive Affinität zu Motilin-Rezeptoren, während sie in motilin-agonistisch wirksamen Dosisbereichen keine praktisch relevante Affinität zu anderen Rezeptoren im gastrointestinalen Trakt wie Adrenalin-, Acetylcholin-, Histamin-, Dopamin- oder Serotonin-Rezeptoren zeigen.

Um eine geregelte Verdauung der eingenommenen Nahrung zu gewährleisten, wirken im gesunden Zustand das autonome Nervensystem und Hormone des gastrointestinalen Traktes zusammen, um eine geregelte Kontraktionstätigkeit des gastrointestinalen Traktes nicht nur direkt nach Nahrungsaufnahme, sondern auch bei leerem gastrointestinalen Trakt zu erzeugen. Motilin ist ein bekanntes gastrointestinales Peptidhormon, welches die Motilität des gastrointestinalen Traktes stimuliert und eine koordinierte Motilität im gesamten gastrointestinalen Trakt im nüchternen Zustand sowie nach Nahrungsaufnahme induziert.

Die Verbindungen der Formel I zeigen motilin-artige physiologische Wirkungen, indem sie als Agonisten für Motilinrezeptoren wirksam werden. So zeigen die Verbindungen der Formel I ausgeprägte stimulierende Wirkungen im Magen-Darm-Bereich und am unteren Speiseröhrensphincter. Sie bewirken insbesondere eine Beschleunigung der Magenentleerung und eine langanhaltende Erhöhung des Ruhe-Tonus des Oesophagus Sphincter. Aufgrund ihres motilin-artigen Wirkungsprofils eignen sich die Substanzen zur Behandlung von Krankheitszuständen, welche mit Motilitätsstörungen im gastrointestinalen Trakt und/oder Rückfluß von Speisebrei aus dem Magen in die Speiseröhre verbunden sind. So sind die Verbindungen der Formel I z.B. indiziert bei Gastroparesis verschiedensten Ursprungs, Störungen der Magenentleerung und gastroösophagalem Rückfluß, Dyspepsie, Anomalien der Colonmotilität, wie sie z.B. bei dem irritablen Colon (= irritable bowel syndrom, abgekürzt IBS) auftreten und postoperativen Motilitätsstörungen, z.B. Darmverschluß (Ileus).

Die gastrointestinal wirksamen Eigenschaften der Verbindungen der Formel I lassen sich in pharmakologischen Standardtestmethoden in vitro und in vivo nachweisen.

### Beschreibung der Testmethoden.

### 1. Bestimmung des Bindungsvermögens der Testssubstanzen an Motilin-Rezeptoren.

Die Affinität der Verbindungen der Formel I an Motilin-Rezeptoren wird in vitro an einer Fraktion eines Gewebehomogenats aus dem Kaninchenantrum gemessen. Bestimmt wird die Verdrängung von radioaktiv markiertem jodiertem Motilin aus der Motilin-Rezeptor-Bindung durch die Testsubstanzen.

Die Rezeptor-Bindungsstudien werden nach einer Modifikation der Methode von Borman et al. (Regulatory Peptides 15 (1986), 143 - 153) durchgeführt. Zur Herstellung des ¹²⁵Jod-markierten Motilins wird Motilin auf an sich bekannte Weise, z.B. analog der von Bloom et al. (Scand. J. Gastroenterol. 11 (1976) 47 - 52) beschriebenen Methode enzymatisch unter Verwendung von Lactoperoxidase jodiert.

Zur Gewinnung der in dem Test verwendeten Gewebehomogenatfraktion aus dem Kaninchenantrum wird das von Schleimhäuten befreite Antrum zerkleinert und im 10-fachen Volumen einer kalten Homogenisierungspufferlösung (50 mM Tris-HCl-Puffer, 250 mM Sucrose, 25 mM KCl, 10 mM MgCl₂, pH 7,4) mit Zusatz von Inhibitoren (1 mM Jodacetamid, 1 µM Pepstatin, 0,1 mM Methylsulfonylfluorid, 0,1 g/l Trypsininhibitor, 0,25 g/l Bacitracin) mit einem Homogenisator 15 sec. bei 1500 Umdrehungen pro Minute homogenisiert. Das Homogenisat wird dann 15 Minuten lang bei 1000 g zentrifugiert, der erhaltene Rückstand wird viermal mit Homogenisierungspufferlösung gewaschen und schließlich in 0,9 %-iger Natriumchloridlösung (in einem der 5-fachen Gewichtsmenge des Antrums entsprechendem Volumen) resuspendiert. Die so erhaltene Gewebefraktion, welche als "rohe Membranzubereitung" bezeichnet wird, wird für den Test eingesetzt.

Für den Bindungsversuch werden 200 µl der rohen Membranfraktion (0,5 - 1 mg Protein) in 400 µl einer Pufferlösung A (50 mM Tris-HCl-Puffer, 1,5 % BSA, 10 mM MgCl₂, pH 8,0) mit 100 µl jodiertem Motilin in Pufferlösung B (10 mM Tris-HCl-Puffer, 1 % BSA, pH 8) verdünnt (Endkonzentration 50 pM) 60 min. bei 30 °C inkubiert. Die Reaktion wird durch Zugabe von 3,2 ml kalter Pufferlösung B gestoppt und gebundenes und nichtgebundenes Motilin werden durch Zentrifugieren (1000 g, 15 Minuten) voneinander getrennt. Der nach dem Zentrifugieren als Pellet erhaltene Rückstand wird mit Pufferlösung B gewaschen und in einem Gamma-Zähler ausgezählt. Die Verdrängungsstudien werden durch Zugabe steigender Mengen der zu testenden Substanz in das Inkubationsmedium durchgeführt. Als Testsubstanzlösungen werden wäßrige Lösungen eingesetzt, welche durch geeignete Verdünnung von 60 x 10⁻⁴-molaren wäßrigen Stammlösungen hergestellt werden. In Wasser schwer lösliche Testsubstanzen werden zunächst in 60 %-igem Äthanol gelöst und diese Lösung wird mit soviel Wasser verdünnt, daß in der zu testenden Lösung die Äthanolkonzentration 1,6 Vol.-% nicht übersteigt. Aus den erhaltenen Meßdaten wird als IC₅₀ der jeweiligen Testsubstanz diejenige Konzentration bestimmt, welche eine 50 %-ige Hemmung der spezifischen Bindung des jodierten Motilin an die Motilin-Rezeptoren bewirkt. Aus dieser wird der entsprechende pIC₅₀-Wert berechnet. Nach der vorstehenden Methode wurde für die Substanz des Beispiels 1 ein pIC₅₀-Wert von 8,32 bestimmt.

### 2. In-vivo-Bestimmung des Einflusses der Substanzen auf die Magenentleerungsgeschwindigkeit.

Die Bestimmung der Magenentleerungsgeschwindigkeit wird an Beagle-Hunden durchgeführt, welchen vor dem Versuch operativ eine Oesophagus-Fistel angelegt und eine Duodenal-Kanüle implantiert wurden. 15 min. nach duodenaler Applikation der Testsubstanzen werden den nüchternen wachen Hunden 285 g einer halbfesten kalorischen Testmahlzeit über die Oesophagus-Fistel verabreicht. Der vom Magen entleerte Inhalt wird in 15-Minuten-Intervallen über die Duodenal-Kanüle aufgefangen. Aus den aufgefangenen Mengen an Mageninhalt wird diejenige Zeitspanne berechnet, innerhalb welcher eine 50 %-ige Entleerung des Magens stattfindet. Die Zeitspanne wird als Maß für die Magenentleerung angegeben.

In diesem Testmodell zeigte die Verbindung des Beispiels 1 eine deutliche Stimulierung der Magenentleerung bei einer Dosis von 0,46 µMol/kg. Die Zeit für eine 50 %-ige Entleerung des Magens verringerte sich von 46 min. bei einer Kontrolltiergruppe auf 27 min. bei Tieren, welche die Testsubstanz erhalten hatten.

### 3. In-vivo-Bestimmung des Einflusses der Substanzen auf den Ruhe-Tonus des Oesophagus Sphincter.

Diese Bestimmung wird an trainierten, wachen, nüchternen Beagle-Hunden vorgenommen, denen vor Versuch je eine Oesophagus-Fistel und eine Duodenal-Kanüle gelegt worden sind. Der Druck des unteren Oesophagus Sphincter wird mittels eines perfundierten Kathetersystems mit seitlicher Öffnung, das mit einem Druckaufnehmer und einem Recorder verbunden ist, gemessen. Der Katheter wird über die Oesophagus-Fistel in den Magen geführt und dann langsam manuell zurückgezogen (= Durchzugsmanometrie). Beim Passieren des Katheterteils mit der seitlichen Öffnung durch die Hochdruckzone des unteren Oesophagus Sphincter wird ein Peak registriert. Aus diesem Peak wird der Druck in mm Hg bestimmt.

Auf diese Weise wird zunächst als Kontrollwert der basale Druck des Oesophagus Sphincter bestimmt. Anschließend wird die Testsubstanz intraduodenal appliziert und nach 15 min. wird der Druck am unteren Oesophagus Sphincter in 2-Minuten-Intervallen über einen Zeitraum von 46 min. gemessen. Die Steigerung des Druckes nach Testsubstanzgabe im Vergleich zu dem vorbestimmten basalen Druck wird berechnet.

In diesem Test wurde der basale Tonus des Oesophagus Sphincter durch eine Dosis von 0,251 µMol/kg der Substanz des Beispiels 1 mehr als verdoppelt. Dieser Effekt hielt während der gesamten Testdauer von 45 min. an.

Aufgrund ihrer Wirkungen im gastrointestinalen Trakt sind die Verbindungen der Formel I in der Gastroenterologie als Arzneimittel für größere Säugetiere, insbesondere Menschen, zur Prophylaxe und Behandlung von Motilitätsstörungen des gastrointestinalen Traktes geeignet.

Die zu verwendenden Dosen können individuell verschieden sein und variieren naturgemäß je nach Art des zu behandelnden Zustandes und der Applikationsform. Zum Beispiel werden parenterale Formulierungen im allgemeinen weniger Wirkstoff enthalten als orale Präparate. Im allgemeinen eignen sich jedoch für Applikationen an größeren Säugetieren, insbesondere Menschen, Arzneiformen mit einem Wirkstoffgehalt von 5 bis 200 mg pro Einzeldosis.

Als Heilmittel können die Verbindungen der Formel I mit üblichen pharmazeutischen Hilfsstoffen in galenischen Zubereitungen wie z.B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe, wie z.B. Milchzucker, Stärke oder Talkum oder flüssiger Verdünnungsmittel, wie z.B. Wasser, fetten Ölen oder flüssigen Paraffinen und unter Verwendung von pharmazeutisch üblichen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

### Beispiel 1:

[2R,3R(2R',3R'),6R,7S,8S,9R,10R]-3-(2',3'-Dihydroxypent-2'-yl)-7-[(2,6-dideoxy-3-C-methyl-3-0-methyl-α-L-ribo-hexopyranosyl)-oxy]-9-[(3,4,6-trideoxy-3-(N-methyl-N-isopropylamino)-β-D-xylohexopyranosyl)-oxy]-2,6,8,10,12-pentamethyl-4,13-dioxabicyclo[8.2.1]tridec-12-en-5-on (= Verbindung der Formel I, R¹ = Methyl).
A) Herstellung von N-Desmethylerythromycin A.
   20 g Erythromycin A (= 27,2 mmol) und 11,2 g (= 136,2 mmol) Natriumacetat wurden in 200 ml eines Gemisches aus Methanol/Wasser 8 : 2 gelöst. Die Lösung wurde auf 47 °C erwärmt. Dann wurden 6,9 g (= 136,2 mmol) Jod zugegeben. Der pH-Wert wurde durch Zugeben von verdünnter wäßriger Natriumhydroxidlösung auf 8 bis 9 gehalten. Nach 3 Stunden wurde das Reaktionsgemisch zur Aufarbeitung in ein Gemisch aus 1 l Wasser und 20 ml Ammoniumhydroxidlösung gegossen. Das Reaktionsgemisch wurde mit Essigsäureethylester extrahiert, und der organische Extrakt mit Ammoniumhydroxid-haltigem Wasser gewaschen und eingeengt. Das nach Entfernen des Lösungsmittels verbleibende Rohprodukt wurde aus Aceton/Ammoniumhydroxidlösung 50 : 3 umkristallisiert. Schmelzpunkt 143 - 148 °C.
B) Herstellung von N-Desmethyl-8,9-anhydroerythromycin-A-6,9-hemiketal (= Verbindung der Formel IV, R¹ = Methyl).
   21 g des unter A) erhaltenen Produktes wurden in 110 ml Eisessig gelöst und die Lösung wurde 1 Stunde bei Raumtemperatur gerührt. Dann wurde das Reaktionsgemisch zur Aufarbeitung unter Eiskühlung in 400 ml konzentrierte Ammoniumhydroxidlösung getropft. Das Reaktionsgemisch wurde mit Essigsäureethylester extrahiert, der organische Extrakt mit Wasser gewaschen und das Lösungsmittel abgezogen. Das als Rückstand verbleibende Rohprodukt wurde erst aus Ether und dann aus Methanol umkristallisiert. Es wurden 14 g reines Produkt mit einem Schmelzpunkt von 145 °C erhalten.
C) Herstellung von [2R,3R(2R',3R'),6R,7S,8S,9R,10R]-3-(2',3'-Dihydroxypent-2'-yl)-7-[(2,6-dideoxy-3-C-methyl-3-0-methyl-α-L-ribo-hexopyranosyl)-oxy]-9-[(3,4,6-trideoxy-3-methylamino-β-D-xylo-hexopyranosyl)-oxy]-2,6,8,10,12-pentamethyl-4,13-dioxabicyclo[8.2.1]tridec-12-en-5-on (= Verbindung der Formel II, R¹ = Methyl).
   9,4 g (= 13,4 mmol) des unter B) erhaltenen Produktes wurden mit 1,9 g (= 13,4 mmol) Kaliumcarbonat in Methanol 2,5 Stunden unter Rückfluß gekocht. Zur Aufarbeitung wurde das Reaktionsgemisch eingeengt, mit Wasser verdünnt und mit Essigsäureethylester extrahiert. Das nach Entfernen des Lösungsmittels verbleibende Rohprodukt wurde aus Isopropanol umkristallisiert. Es wurden 7,1 g reines Produkt mit einem Schmelzpunkt von 199 bis 200 °C erhalten, optischer Drehwert [α]²⁰_{D} :
   - 31,6 ° (c = 1, Methanol).
D) Herstellung der Titelverbindung.
   2 g (= 2,8 mmol) des vorstehend unter C) erhaltenen Produktes wurden in Methanol gelöst und der pH-Wert der Lösung wurde durch Zugabe von verdünnter Salzsäurelösung auf 4 eingestellt. Zu der Lösung wurden 2 g eines Molekularsiebs (Calciumaluminiumsilikat, Porendurchmesser 4 Å), ein Überschuß an Aceton und 0,4 g (= 6,4 mmol) Natriumcyanoborhydrid gegeben. Das Reaktionsgemisch wurde 12 Stunden gerührt. Zur Aufarbeitung wurde von dem Molekularsieb abfiltriert, das Filtrat eingeengt, mit Wasser versetzt und mit Essigsäureethylester extrahiert. Das nach Einengen des Essigsäureethylesterextraktes als Rückstand verbleibende Rohprodukt wurde säulenchromatographisch über Kieselgel (Elutionsmittel Essigsäureethylester/Methanol 95 : 5) gereinigt. Es wurden 1,4 g der Titelverbindung mit einem Schmelzpunkt von 130 bis 134 °C erhalten, optischer Drehwert [α]²⁰_{D} : - 32,8 °.

### Beispiel 2:

[2R,3R(2R',3R'),6R,7S,8S,9R,10R]-3-(2',3'-Dihydroxypent-2'-yl)-7-[(2,6-dideoxy-3-C-methyl-3-0-methyl-α-L-ribo-hexopyranosyl)-oxy]-9-[(3,4,6-trideoxy-3-(N-methyl-N-isopropylamino)-β-D-xylohexopyranosyl)-oxy]-2,6,8,10,12-pentamethyl-4,13-dioxabicyclo[8.2.1]tridec-12-en-5-on(= Verbindung der Formel I, R¹ = Methyl).
A) Herstellung N-Desmethylerythromycin A.
   5 g Erythromycin A und 4,7 g Natriumacetat (x 3 H₂O) wurden in 200 ml eines Gemisches aus Methanol/Wasser 8:2 gelöst. Zu der Lösung wurden 1,75 g Jod zugegeben und das Reaktionsgemisch wurde sodann bei Raumtemperatur 20 Minuten lang mit einer Quarzlampe bestrahlt. Anschließend wurde die Hälfte des Lösungsmittels abgedampft, und das verbleibende Reaktionsgemisch wurde in ein Gemisch aus 140 ml Wasser und 10 ml Ammoniak gegossen. Das Reaktionsgemisch wurde mit dreimal 20 ml Methyl-t-butylether extrahiert. Der Etherextrakt wurde abgetrennt und der Ether teilweise abgedampft. Dann wurde das Reaktionsprodukt auskristallisieren gelassen und aus Aceton umkristallisiert. Es wurden 2 g N-Desmethylerythromycin A erhalten.
B) Zur Herstellung von N-Desmethyl-8,9-anhydroerythromycin-A-6,9-hemiketal (= Verbindung der Formel IV, R¹ = Methyl) wurden 2 g des unter A) erhaltenen Produktes wie in Beispiel 1 B) beschrieben behandelt. Es wurden 2,3 g des Hemiketals als amorpher Feststoff erhalten.
C) Zur Herstellung von [2R,3R(2R',3R'),6R,7S,8S,9R,10R]-3-(2',3'-Dihydroxypent-2'-yl)-7-[(2,6-dideoxy-3-C-methyl-3-0-methyl-α-L-ribo-hexopyranosyl)-oxy]-9-[(3,4,6-trideoxy-3-methylamino-β-D-xylo-hexopyranosyl)-oxy]-2,6,8,10,12-pentamethyl-4,13-dioxabicyclo[8.2.1]tridec-12-en-5-on (= Verbindung der Formel II, R¹ = Methyl) wurden 2,3 g des vorstehend erhaltenen Produktes wie in Beispiel 1 C) beschrieben behandelt. Das erhaltene Rohprodukt wurde aus Ethylacetat umkristallisiert. Es wurden 1,3 g reines Produkt mit einem Schmelzpunkt von 199 - 202 °C erhalten.
D) 1,3 g des vorstehend erhaltenen Produktes wurden in ein Gemisch aus 26 ml Aceton und 0,11 ml Essigsäure gegeben. Zu dem Reaktionsgemisch wurden 0,6 g Natriumtriacetoxyborhydrid portionsweise unter Stickstoffatmosphäre zugegeben und das Reaktionsgemisch wurde 4 Stunden bei Raumtemperatur gerührt. Dann wurden zwei Drittel des Lösungsmittels abgedampft, und der Rückstand wurde mit 40 ml Ethylacetat verdünnt. Unter kräftigem Rühren wurden 65 ml einer gesättigten Natriumhydrogencarbonatlösung zugegeben. Von der sich bildenden klaren Lösung wurde die organische Phase abgetrennt und die wäßrige Phase wurde nochmals mit 20 ml Ethylacetat gewaschen. Die vereinigten organischen Phasen wurden mit 13 ml Wasser gewaschen und über Natriumsulfat getrocknet. Das Lösungsmittel wurde abgedampft, der Rückstand wurde in 20 ml Toluol aufgenommen und dieses dann wiederum abgedampft. Das erhaltene Rohprodukt wurde durch Filtration über eine Aluminiumoxidsäule (25 g Al₂O₃, Aktivitätsstufe II/III) unter Verwendung von Ethylacetat als Elutionsmittel gereinigt. Dann wurde das Lösungsmittel abgedampft und der Rückstand in siedendem Ethylacetat gelöst. Anschließend wurde n-Hexan zugegeben bis die Mischung sich trübte. Man ließ das Produkt in der Kälte auskristallisieren. Die gebildeten Kristalle wurden unter vermindertem Druck abfiltriert und mit n-Hexan gewaschen. Es wurden 0,8 g der Titelverbindung mit einem Schmelzpunkt von 128 - 135 °C erhalten.
E) Zur Überführung in ihr Acetat wurden 1 g (= 1,3 mmol) der Titelverbindung in Methanol gelöst und die Lösung wurde mit 0,08 ml (= 1,3 mmol) Eisessig versetzt. Anschließend wurde das Lösungsmittel unter vermindertem Druck abgezogen und das gebildete Acetat der Titelverbindung getrocknet. Schmelzpunkt des Acetates der Titelverbindung: 145 - 150 °C
   Optischer Drehwert [α]²⁰_{D}: -30,8 ° (c = 1, Methanol).

### Beispiel I:

Man stellte Tabletten in folgender Zusammensetzung pro Tablette her:

| | |
|---|---|
| [2R,3R(2R',3R'),6R,7S,8S,9R,10R]-3-(2',3'-Dihydroxypent-2'-yl)-7-[(2,6-dideoxy-3-C-methyl-3-0-methyl-α-L-ribo-hexopyranosyl)-oxy]-9-[(3,4,6-trideoxy-3-(N-methyl-N-isopropylamino)-β-D-xylo-hexopyranosyl)-oxy]-2,6,8,10,12-pentamethyl-4,13-dioxabicyclo[8.2.1]tridec-12-en-5-on (= Verbindung der Formel I, R¹ = Methyl) | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker wurden mit der 10%-igen Gelatine-Lösung eingedickt. Die Paste wurde zerkleinert und das entstandene Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

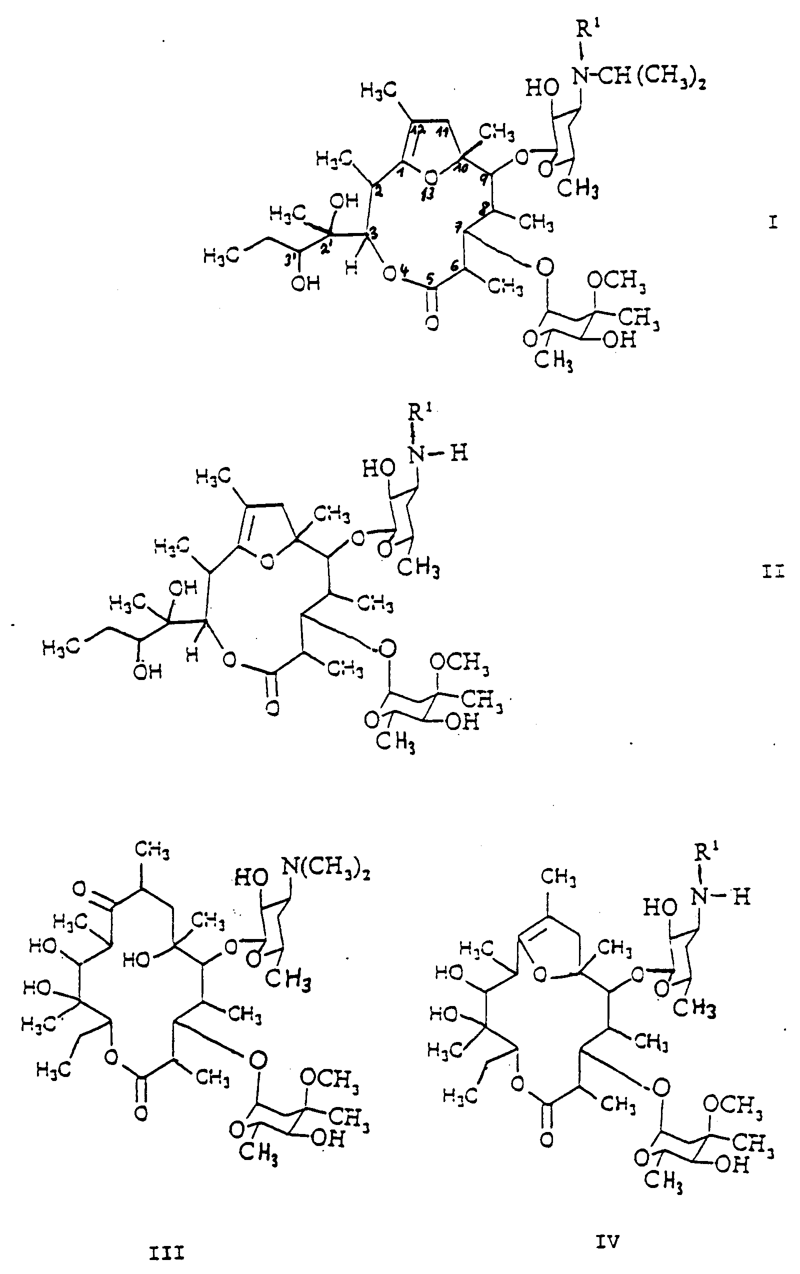

## Patentansprüche

1. [2R,3R(2R',3R'),6R,7S,8S,9R,10R]-3-(2',3'-Dihydroxypent-2'-yl)-2,6,8,10,12-pentamethyl-4,13-dioxabicyclo[8.2.1]tridec-12-en-5-on-Derivate der allgemeinen Formel I 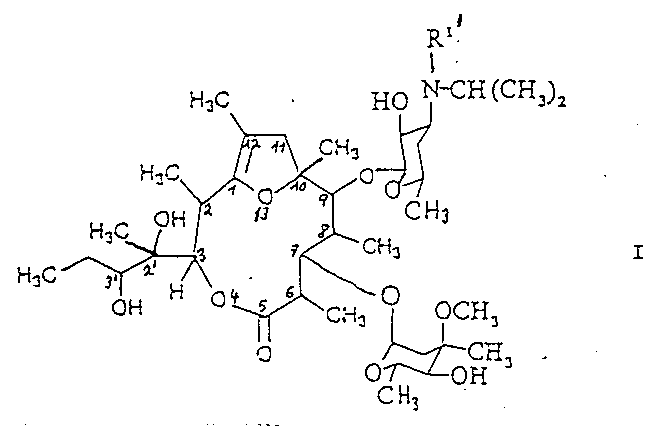 worin R^{1'} Methyl bedeutet, und deren stabile und physiologisch verträgliche Säureadditionssalze.

2. Arzneimittel, enthaltend eine pharmakologisch wirksame Menge einer Verbindung gemäß Anspruch 1 und übliche pharmazeutische Hilfs- und/oder Trägerstoffe.

3. Verfahren zur Herstellung von [2R,3R(2R',3R'),6R,7S, 8S,9R,10R]-3-(2',3'-Dihydroxypent-2'-yl)-2,6,8,10,12-pentamethyl-4,13-dioxabicyclo[8.2.1]tridec-12-en-5-on-Derivate der allgemeinen Formel I, 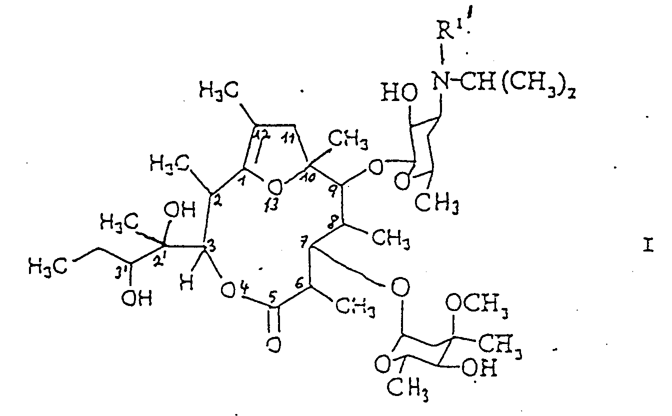 worin R^{1'} Methyl bedeutet, und deren stabilen und physiologisch verträglichen Säureadditionssalzen, dadurch gekennzeichnet, daß man in [2R,3R(2R',3R'),6R,7S,8S,9R, 10R]-3-(2',3'-Dihydroxypent-2'-yl)-2,6,8,10,12-pentamethyl-4,13-dioxabicyclo[8.2.1]tridec-12-en-5-on-Derivate der allgemeinen Formel II 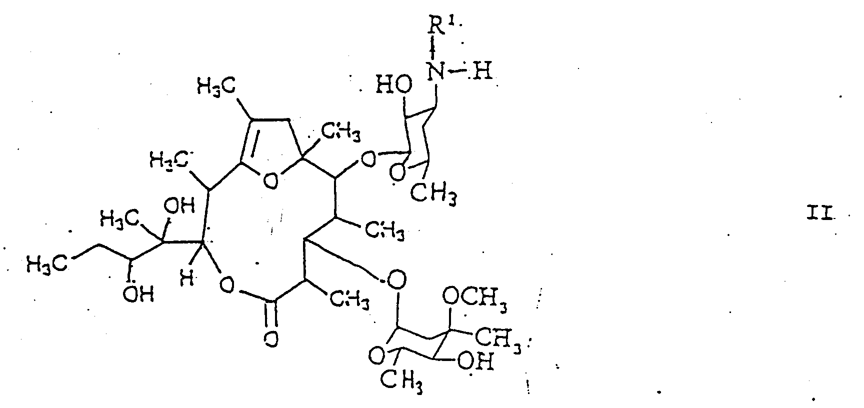 worin R¹ Wasserstoff oder Methyl bedeutet,
einen Isopropylrest einführt,
und in einer erhaltenen Verbindung, worin R¹ Wasserstoff bedeutet, einen Methylrest R^{1'} einführt und gewünschtenfalls freie Verbindungen der Formel I in ihre stabilen Säureadditionssalze überführt oder die Säureadditionssalze in die freien Verbindungen der Formel I überführt.

## Claims

1. [2R,3R(2R',3R'),6R,7S,8S,9R,10R]-3-(2',3'-dihydroxypent-2'-yl)-2,6,8,10,12-pentamethyl-4,13-dioxabicyclo[8.2.1]tridec-12-en-5-one derivatives of the general formula I 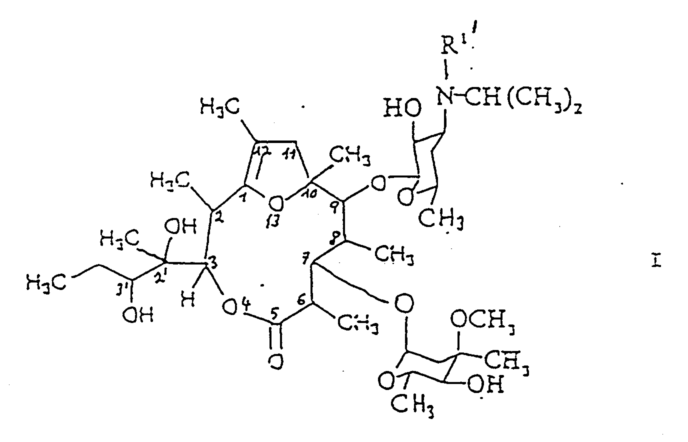 in which R^{1'} denotes methyl, and the stable and physiologically tolerated acid addition salts thereof.

2. Pharmaceutical containing a pharmacologically effective amount of a compound according to Claim 1 and conventional pharmaceutical auxiliary substances and/or vehicles.

3. Process for the preparation of [2R,3R(2R',3R'),6R,7S,8S,9R,10R]-3-(2',3'-dihydroxypent-2'-yl)-2,6,8,10,12-pentamethyl-4,13-dioxabicyclo[8.2.1]tridec-12-en-5-one derivatives of the general formula I 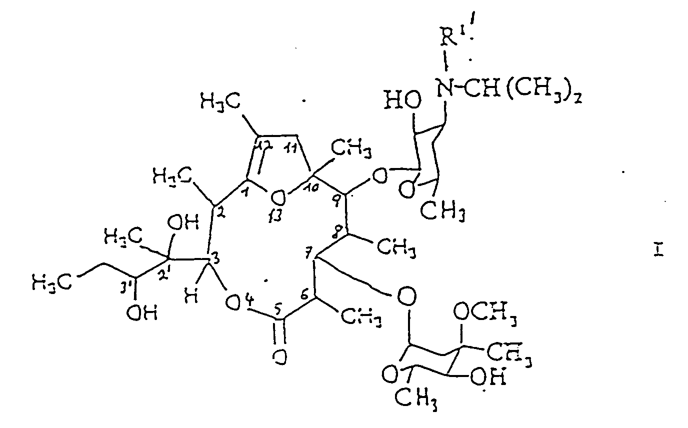 in which R^{1'} denotes methyl, and the stable and physiologically tolerated acid addition salts thereof, characterised in that an isopropyl radical is introduced into [2R,3R(2R',3R'),6R,7S,8S,9R,10R]-3-(2',3'-dihydroxypent-2'-yl)-2,6,8,10,12-pentamethyl-4,13-dioxabicyclo[8.2.1]tridec-12-en-5-one derivatives of the general formula II 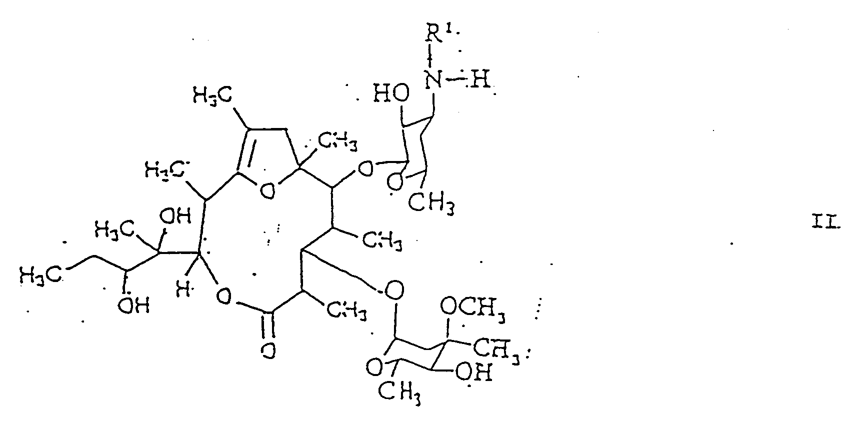 in which R¹ is hydrogen or methyl,
and a methyl radical R^{1'} is introduced into the resulting compound in which R¹ denotes hydrogen,
and, if desired, free compounds of the formula I are converted into their stable acid addition salts, or the acid addition salts are converted into the free compounds of the formula I.

## Revendications

1. Dérivés de [2R,3R(2R',3R'),6R,7S,8S,9R,10R]-3-(2'-3'-dihydroxypent-2'-yl)-2,6,8,10,12-pentaméthyl-4,13-dioxabicyclo[8.2.1]tridéc-12-ène-5-one de formule générale I : 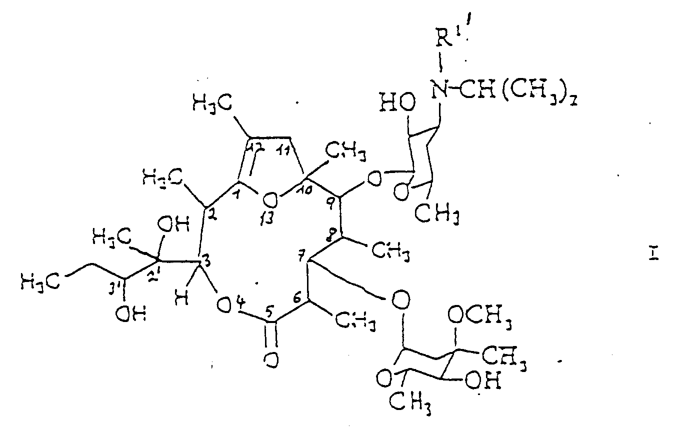 dans laquelle R^{1'} désigne un groupe méthyle, et leurs sels d'addition avec des acides, stables et physiologiquement compatibles.

2. Médicament contenant une quantité pharmacologiquement efficace d'un composé selon la revendication 1 et des adjuvants et/ou des véhicules pharmaceutiques usuels.

3. Procédé de préparation de dérivés de [2R,3R(2R',3R'),6R,7S,8S,9R,10R]-3-(2'-3'-dihydroxypent-2'-yl)-2,6,8,10,12-pentaméthyl-4,13-dioxabicyclo[8.2.1] tridéc-12-ène-5-one de formule générale I : 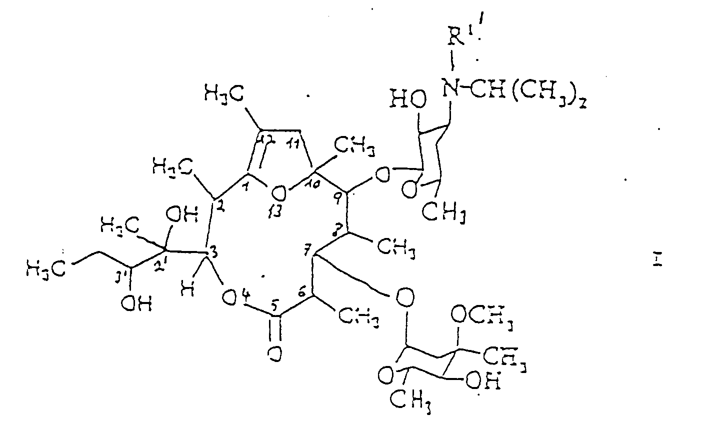 dans laquelle R^{1'} désigne un groupe méthyle, et leurs sels d'addition avec des acides, stables et physiologiquement compatibles, caractérisé en ce qu'on introduit un radical isopropyle dans des dérivés de [2R,3R(2R',3R'), 6R,7S,8S,9R,10R]-3-(2'-3'-dihydroxypent-2'-yl)-2,6,8,10,12-pentaméthyl-4,13-dioxabicyclo[8.2.1] tridéc-12-ène-5-one de formule générale II : 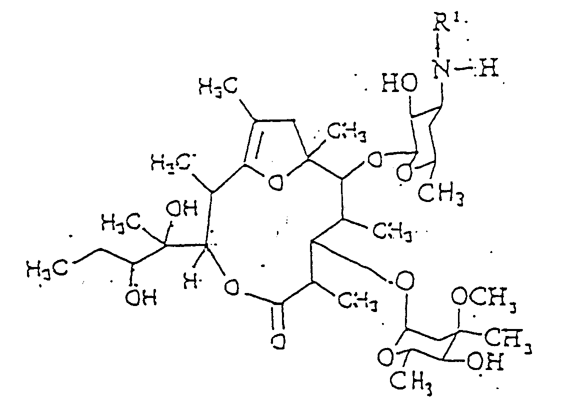 dans laquelle R¹ désire de l'hydrogène ou un groupe méthyle,
et on introduit un radical méthyle R^{1'} dans le composé obtenu, dans lequel R¹ désigne de l'hydrogène, et, si on le souhaite, on convertit les composés libres de formule I en leurs sels stables d'addition avec des acides ou les sels d'addition avec des acides en composés libres de formule I.
